Europäisches Patentamt

European Patent Office

Office européen des brevets

(11) Publication number: **0 306 455**
**A1**

## (12) EUROPEAN PATENT APPLICATION

(21) Application number: **88810586.3**

(22) Date of filing: **25.08.88**

(51) Int. Cl.4: **A 61 K 31/155**
**A 61 K 9/18**

(30) Priority: **31.08.87 US 91237**

(43) Date of publication of application:
**08.03.89 Bulletin 89/10**

(84) Designated Contracting States:
**BE DE ES FR GB GR**

(71) Applicant: **WARNER-LAMBERT COMPANY**
**201 Tabor Road**
**Morris Plains New Jersey 07950 (US)**

(72) Inventor: **Gallopo, Andrew Robert**
**133, Wessington Avenue**
**Garfield, NJ 07026 (US)**

**Lynch, Donald Michael**
**19 Fieldstone Place**
**Flemington, NJ 08822 (US)**

(74) Representative: **Silbiger, Jakob, Dr.**
**c/o CAPSUGEL AG Münchensteinerstrasse 41**
**CH-4002 Basel (CH)**

(54) **Cyclodextrin complexes of bis-biguanido hexane compounds.**

(57) The invention provides cyclodextrin complexes of bis-biguanido hexane compounds such as chlorhexidine and its salts which act to increase the water solubility of the chlorhexidine or its salts, mask their bitter after taste and increase their bio-availability.

The invention also provides aqueous-based anti-bacterial oral preparations containing such complexes which may also contain colorants, flavorants, sweeteners, fluorides and polishing agents.

EP 0 306 455 A1

**Description**

## CYCLODEXTRIN COMPLEXES OF BIS-BIGUANIDO HEXANE COMPOUNDS

This invention is concerned with cyclodextrin complexes of bis-biguanido hexane compounds such as chlorhexidine and an anti-bacterial oral composition containing such cyclodextrin complexes.

Bis-biguanido hexane compounds such as chlorhexidine and its salts are well known in the art for their anti-bacterial activity and have been used in aqueous-based oral compositions to counter dental plaque and caries formation by bacteria in the oral cavity. Chlorhexidine is active against a wide range of gram-positive and gram-negative organisms, yeast, fungi, facultative anaerobes and aerobes.

Chlorhexidine is a strong base and is most stable in the form of salts such as the digluconate and dihydrochloride. The most commonly used derivative is the digluconate salt because of its high water-solubility. Chlorhexidine dihydrochloride has low water solubility, i.e. on the order of 60 mg per 100 ml of water. Free chlorhexidine also exhibits low water solubility, i.e. on the order of 8 mg. per 100 ml. of water.

Chlorhexidine and its derivatives also have the disadvantage that they stain the teeth with repeated use and have a bitter taste.

Thus, it would be commercially advantageous to increase the water solubility of chlorhexidine and its derivatives and to mask the bitter taste of these compounds so that they can be more readily incorporated into aqueous based compositions and find greater acceptability to the user.

Cyclodextrins have long been known to form inclusion complexes with various compounds. The cyclodextrin molecule comprises a plurality of glucopyranose units arranged in a torus-like configuration having all the secondary hydroxy groups located on one side of the torus and all primary hydroxyl groups located on the other side. Alpha, beta, and gamma cyclodextrin contain 6, 7 & 8 cyclic glucopyranose units, respectively, in the torus shell. The "lining" of the internal cavity is formed by hydrogen and glucosidic oxygen-bridge atoms and therefore the surface is slightly apolar.

Molecules, or functional groups of molecules having molecular dimensions that match the cyclodextrin cavity, being less hydrophilic than water, can be included into the cyclodextrin cavity in the presence of water. In aqueous solution the slightly apolar cyclodextrin cavity is occupied by water molecules which are energetically unfavored (polar-apolar interaction) and are therefore readily substituted by appropriate "guest molecules" which are less polar than water.

Almost all industrial applications of cyclodextrins involve complexation and include complexation of flavors, fragrances, drugs and pesticides to protect them against the action of light, oxygen and the loss of volatile constituents.

Heretofore, bis-biguanido hexane compounds such as chlorhexidine have not been complexed with cyclodextrins.

Chlorhexidine has, however, been used as a preservative for complexes of cyclodextrin and agents such as polypeptides, polysaccharides, aminoglycosides, anti-biotics, beta-lactose, anti-biotics, nucleic acids, water-soluble drugs and lipophilic drugs. See Japanese Patent No. 60149530.

The present invention, however, provides cyclodextrin complexes of bis-biguanido hexane compounds, particularly chlorhexidine and its derivatives which act to increase the solubility of the chlorhexidine or derivative making such compounds easier to incorporate in aqueous-based anti-bacterial compositions. Further, by virtue of complexing the bitter taste of the chlorhexidine is effectively masked. In addition, it has been found that the bio-availability of the chlorhexidine can be increased by complexing with cyclodextrins.

The invention provides cyclodextrin complexes of bis-biguanido hexane compounds, particularly chlorhexidine and its salts which are formed by dissolving a molar excess of cyclodextrin in deionized water at elevated temperatures and adding thereto chlorhexidine or its salts. The solution is cooled, frozen and lyophilized to obtain the complex. Complex formation was confirmed by NMR analysis.

The cyclodextrin complexes act to increase the water solubility of the corresponding chlorhexidine or chlorhexidine salt from about 6 to 10 times rendering them more easily incorporated into aqueous-based compositions. The complexes also mask the bitter aftertaste of chlorhexidine and enhance their acceptability in oral preparations. The complexes do not affect chlorhexidine's anti-microbial activity and even act to increase its bio-availability.

The invention also provides aqueous-based anti-bacterial oral preparations containing these complexes which may also contain flavorants, colorants, sweeteners, fluorides and polishing agents.

The preferred bis-biguanido hexanes which may be employed in the present invention include chlorhexidine, 1,6- di(p-chlorophenylbiguanides) hexane, and the acid addition salts of chlorhexidine, particularly the digluconate and dihydrochloride. However, any non-toxic, anti-bacterial, water-soluble salt of a bis-biguanido hexane may be employed such as the diacetate, dihydrogen halides such as the difluoride and dibromide, diamino fluorophosphate and the like.

The cyclodextrins, according to the invention include alpha, beta and gamma cyclodextrin containing 6, 7 and 8 cyclic glucopyranose units, respectively, in the torus shell. Beta-cyclodextrin is preferred due to its availability and efficiency in forming complexes.

The cyclodextrin complexes according to the invention are formed by first dissolving cyclodextrin in de-ionized water and heating the solution to temperatures of from about 65° to 85°, preferably about 75°C, and then adding the chlorhexidine compound with mixing until a complex is formed, generally from about 30 seconds to 10 minutes. The cyclodextrin should be present in a molar excess with respect to the chlorhexidine compound, on the order of 0.25 to 0.75, preferably a 0.5 molar excess. The mixture is then cooled to room temperature, and may be used as is or frozen and lyophilized to a white solid. Evidence of the formation of the complex has been shown by NMR and increased water-solubility of the complex. It has been shown that by complexing with cyclodextrins, chlorhexidine and its salts exhibit increased solubility. For example, chlorhexidine dihydrochloride is soluble in water to the extent of 60 mg. per 100 ml. at room temperature. When complexed with cyclodextrin the solubility increases to 576 mg. per 100 ml., nearly a ten-fold enhancement. Similarly, uncomplexed chlorhexidine as the free base is soluble to the extent of 8 mg. per 100 ml. of water at room temperature, but as the cyclodextrin complex it increases to 58 mg. per 100 ml., a seven-fold solubility increase. This increased solubility is further evidence of complex formation.

Complexes according to the invention also mask the bitter taste of such chlorhexidine compounds as chlorhexidine digluconate.

Also, it has been found, that not only do the cyclodextrin complexes of chlorhexidine compounds not affect the anti-microbial activity of the chlorhexidine but also increase its bio-availability. In tests for bio-availability comprising chlorhexidine digluconate and its cyclodextrin complex a 0.12% aqueous solution of chlorhexidine as the complex demonstrated an antimicrobial activity equivalent to a 0.23% solution of chlorhexidine as the digluconate alone.

The cyclodextrin complexes of the invention can be readily incorporated into aqueous or aqueous-alcohol based oral preparations such as a mouthwash, spray, rinse, toothpaste, dental cream or toothpowder.

The complex should be present in amounts of from about 0.01% to about 10% by weight of the total weight of the preparation. Preferably the complex is present in amounts from about 0.05% to about 1% by weight of the total weight and most preferably from about 0.05% to about 0.5%.

In one form of the invention, the oral preparation may be a liquid such as a mouthwash, spray or rinse. In such a preparation the vehicle is typically a water-alcohol mixture. Generally the ratio of total water to alcohol is in the range of from about 1:1 to about 20:1, preferably about 3:1 to about 20:1 and most preferably about 3:1 to about 10:1 by weight. The total amount of water-alcohol mixture in a mouthwash preparation is typically in the range from about 45% to about 82.5% by weight of the composition. The pH value of such mouthwash preparations is generally from about 4 to about 9 and preferably from about 5 to about 7. A pH below 4 is irritating to the oral cavity and a pH greater than 9 results in an unpleasant mouth feel.

Fluorine providing compounds may be present in the oral preparations of this invention. These compounds may be slightly water soluble or may be fully water soluble and are characterized by their ability to release fluoride ions or fluoride containing ions in water. Typical fluorine providing compounds are inorganic fluoride salts such as soluble alkali metal, alkaline earth metal, and heavy metal salts, for example, sodium fluoride, potassium fluoride, ammonium fluoride, cuprous fluoride, zinc fluoride, stannic fluoride, stannous fluoride, barium fluoride, sodium fluorosilicate, ammonium fluorosilicate, sodium fluorozirconate, sodium monofluorophosphate, aluminum mono- and difluorophosphate and fluorinated sodium calcium pyrophosphate.

Alkali metal, tin fluoride and monofluorophosphates such as sodium and stannous fluoride, sodium monofluorophosphate and mixtures thereof are preferred.

In an oral liquid preparation such as a mouthwash, the fluorine providing compound is generally present in an amount sufficient to release up to about 0.15%, preferably about 0.001% to about 0.1% and most preferably from about 0.001% to about 0.05% fluoride by weight of the preparation.

The oral preparation may also contain additional flavorants and colorants.

In the instance where auxiliary sweeteners are utilized, the present invention contemplates the inclusion of those sweeteners well known in the art, including both natural and artificial sweeteners. Thus, additional sweeteners may be chosen in minor amounts from the following non-limiting list provided they do not inactivate the chlorhexidine compound.

A. Water-soluble sweetening agents such as monosaccharides, disaccharides and polysaccharides such as xylose, ribose, glucose, mannose, galactose, fructose, dextrose, sucrose, maltose, partially hydrolyzed starch or corn syrup solids and sugar alcohols such as sorbitol, xylitol, mannitol and mixtures thereof.

B. Water-soluble artificial sweeteners such as the soluble cyclamate salts and the like.

C. Dipeptide based sweeteners such as L-phenylalanine methyl ester and materials described in U.S. Patent No. 3,492,131 and the like.

In general, the amount of sweetener will vary with the desired amount of sweeteners selected for a particular oral preparation. This amount will normally be 0.01% to about 40% by weight. The water-soluble sweeteners described in category A above, are preferably used in amounts of about 5% to about 40% by weight, and most preferably from about 10% to about 20% by weight of the final composition. In contrast, the artificial sweeteners described in categories B and C are used in amounts of about 0.005% to about 5.0% and most preferably about 0.05% to about 2.5% by weight of the final composition. These amounts are ordinarily necessary to achieve a desired level of sweetness independent from the flavor level achieved from flavorants.

Suitable flavorings include both natural and artificial flavors, and mints such as peppermint and spearmint. Citrus flavors such as orange and lemon, various fruit flavors, both individual and mixed, and the like are contemplated. Aldehyde-containing flavors are to be avoided as aldehydes generally react with chlorhexidine to form Schiff bases. The flavorings are generally utilized in amounts that will vary depending upon the individual flavor, and may, for example, range in amounts of about 0.05% to about 6% by weight of the final composition.

The colorants useful in the present invention include the pigments which may be incorporated in amounts of up to about 2% by weight of the composition. Also, the colorants may include other dyes suitable for food, drug and cosmetic applications, known as FD & C and D & C dyes. The materials acceptable for the foregoing spectrum of use are preferably water-soluble. Illustrative examples include the yellow dye, known as D & C Yellow # 10, and the dye known as FD & C Green # 3 which comprises a triphenylmethane dye. A full recitation of all FD & C and D & C colorants useful in the present invention and their corresponding chemical structures may be found in the Kirk-Othmer Encyclopedia of Chemical Technology, 3rd Edition, in Volume 6, at pages 561-595, which text is accordingly incorporated herein by reference.

The oral preparations may also be substantially solid or pasty in character such as a dental cream, toothpaste or a toothpowder. Solid or pasty oral preparations contain polishing materials. Typical polishing materials are abrasive particulate materials having particle sizes of up to about 20 microns. Nonlimiting illustrative examples include: water-insoluble sodium metaphosphate, potassium metaphosphate, tricalcium phosphate, dihydrated calcium phosphate, calcium pyrophosphate, magnesium orthophosphate, trimagnesium phosphate, calcium carbonate, alumina, aluminum silicate, zirconium silicates, silica, bentonite, and mixtures thereof. Polishing materials are generally present in an amount from about 20% to about 82% by weight of the oral preparation. Preferably, they are present in amounts from about 20% to about 75% in toothpaste, and from about 70% to about 82% in toothpowder. For toothpaste and dental creams the water content is about 25% to 50% by weight.

In clear gels, a polishing agent of colloidal silica and alkali metal aluminosilicate complexes are preferred since they have refractive indicies close to the refractive indicies of gelling agent liquid systems commonly used in dentifrices.

In general. the anti-bacterial oral compositions of the present invention are prepared as follows. The sweetener is dissolved in water to form a solution. The cyclodextrin complex is added to the solution and mixed until dissolved. Then sufficient water, alcohol or mixtures thereof are added with mixing until the final solution volume is reached. When colorants, additional sweeteners and similar additives are included in the composition, they are added at the same time the sweetener is added. In a preferred embodiment the complex is added as the final ingredient.

The compositions of the present invention are prepared at ambient temperatures. Heated solutions are to be avoided as they may cause degradation and loss of some of the components.

The present invention is further illustrated by the following examples.

## EXAMPLES

The following procedure was employed to prepare the following cyclodextrin complexes of chlorhexidine, chlorhexidine digluconate and chlorhexidine dihydrochloride.
    A. Alpha, beta and gamma cyclodextrin complexes of chlorhexidine dihydrochloride.
    B. Beta cyclodextrin complex of chlorhexidine digluconate.
    C. Beta cyclodextrin complex of chlorhexidine free base.

In each case 1.5 millimoles of alpha, beta or gamma cyclodextrin was dissolved in 100 ml. of deionized water and heated to 75°C in a flask. The chlorhexidine compound (1.0 millimoles) is added to the flask with stirring for about 5 minutes. The mixture is then cooled to room temperature quickly by immersion of the flask in an ice water bath. The resulting solution is then frozen and lyophilized to a white solid.

Complex formation was confirmed by NMR analysis. Uncomplexed chlorhexidine dihydrochloride and chlorhexidine digluconate were subjected to 200 megahertz NMR analysis. Each showed an AB spectrum consisting of 2 doublets at 7.3 ppm. Cyclodextrin complexes of these chlorhexidine complexes showed an unsplit singlet at 7.3 ppm showing a change in environment of the chlorhexidine aromatic protons indicating complex formation.

### A. Increased Water Solubility

A normal water solubility of chlorhexidine dihydrochloride is 60 mg. per 100 ml. of water. The alpha, beta and gamma cyclodextrin complexes of chlorhexidine dihydrochloride had a water solubility at room temperature of 576 mg. in 100 ml. of water which amounts to nearly a ten-fold enhancement.

Free chlorhexidine has a water solubility of about 8 mg. per 100 ml. of water at room temperature. The beta-cyclodextrin complex of free chlorhexidine has a water solubility of 58 mg. per 100 ml. of water which amounts to a six-fold increase in solubility.

Since chlorhexidine digluconate is highly water soluble its enhancement as the complex was not measured.

### B. Taste Masking

Aqueous solutions were prepared containing 0.12% of chlorhexidine digluconate and the equivalent quantity of the beta-cyclodextrin complex of chlorhexidine digluconate. Four human subjects took 20 ml. of each solution and rinsed the oral cavity for 10 seconds, then expectorated the solution. Three subjects indicated that the cyclodextrin complexed chlorhexidine had a less bitter aftertaste than the chlorhexidine alone. The fourth indicated no difference in aftertaste.

## C. Affect on Anti-microbial Activity

A comparison of the anti-microbial activity of chlorhexidine digluconate and beta-cyclodextrin complexed chlorhexidine digluconates were compared against four microorganisms, C.albicans, S. anaguis, S.mutans and A.viscosus. The minimum inhibitory concentration (MIC) and minimum lethal concentration (MLC) were found to be similar for both compounds indicating that complex formation does not inhibit the anti-microbial activity of chlorhexidines.

## D. Increased Bio-Availability

Using another test, an agar diffusion assay, it was found that a 0.12% aqueous solution of the beta-cyclodextrin complex of chlorhexidine digluconate demonstrated anti-microbial activity equivalent to a 0.23% solution of chlorhexidine digluconate alone. This result shows an increased bio-availability of the digluconate possibly due to increased solubility in the agar medium.

## Claims

1. A composition of matter comprising a cyclodextrin complex of a bis-biguanido hexane compound.

2. The composition of claim 1 wherein said bis-biguanido hexane compound is selected from the group consisting of chlorhexidine, chlorhexidine digluconate and chlorhexidine dihydrochloride.

3. The composition of the claim 1 or 2 wherein said cyclodextrin is selected from the group consisting of alpha-cyclodextrin, beta-cyclodextrin and gamma-cyclodextrin.

4. A composition of matter according to claim 1 comprising an alpha-cyclodextrin complex of chlorhexidine dihydrochloride.

5. A composition of matter according to claim 1 comprising a beta-cyclodextrin complex of a chlorhexidine selected from the group consisting of chlorhexidine dihydrochloride, chlorhexidine digluconate and chlorhexidine.

6. A composition of matter according to claim 1 comprising a gamma-cyclodextrin complex of chlorhexidine dihydrochloride.

7. A method of preparing a cyclodextrin complex of a chlorhexidine compound as claimed in anyone of the claims 1 to 6 comprising:

    (a) forming an aqueous solution of cyclodextrin at an elevated temperature; and

    (b) adding said chlorhexidine compound to said solution.

8. The method of claim 7 which further comprises cooling said solution.

9. The method of claim 8 which further comprises freezing said solution.

10. The method of claim 9 which further comprises lyophilizing said solution to recover said complex.

11. The method of claim 7 wherein said elevated temperature is between 65° and 85°C and preferably is 75°C.

12. The method of claim 7 wherein said cyclodextrin is present in said solution in a molar excess of from about 0.25 to about 0.75 of said chlorhexidine compound.

13. An aqueous based oral preparation containing a cyclodextrin complex of a bis-biguanido hexane compound as claimed in anyone of the claims 1 to 6.

14. The oral preparation of claim 13 wherein said complex comprises from 0.01% to 10% by weight of the total weight of said preparation, preferably from 0.05% to 1% by weight of the total weight of said preparation, and most preferably from 0.05% to 0.5% of the total weight of said preparation.

15. The oral preparation of claim 13 which further comprises alcohol.

16. The oral preparation according to anyone of the claims 13 to 15 which further comprises an additive selected from the group consisting of a fluorine providing compound, a colorant, a flavorant, an artificial sweetener, a natural sweetener, a polishing agent and mixtures thereof.

17. An aqueous based oral formulation according to anyone of the claims 13 to 16, in the form of a mouthwash, spray, rinse, toothpaste, dental cream or toothpowder.

European Patent Office

# EUROPEAN SEARCH REPORT

Application Number

EP 88 81 0586

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | CHEMICAL ABSTRACTS, vol. 104, 1986, page 320, no. 10610d, Columbus, Ohio, US; & JP-A-60 149 530 (TAKEDA CHEMICAL INDUSTRIES, LTD) 07-08-1985 * Abstract * | 1-4,7, 11,15 | A 61 K 31/155 A 61 K 9/18 |
| A | EP-A-0 094 157 (TAKEDA CHEMICAL INDUSTRIES) * Page 17, lines 7-14; claims 1-10 * | | |
| A | EP-A-0 149 197 (JANSSEN PHARMACEUTICA N.V.) * Claims 1-15 * | | |

TECHNICAL FIELDS SEARCHED (Int. Cl.4)

A 61 K
C 08 B

The present search report has been drawn up for all claims

| Place of search | Date of completion of the search | Examiner |
|---|---|---|
| THE HAGUE | 11-10-1988 | TZSCHOPPE,D.A. |